# EUROPEAN PATENT APPLICATION

(11) **EP 4 353 744 A1**
(43) Date of publication of application: **17.04.2024**
(21) Application number: 22819664.8
(22) Date of filing: 13.06.2022
(51) Int. Cl.: C07K 16/00, C07K 16/08, C07K 16/10, A61K 39/395, A61K 39/42, G01N 33/569, G01N 33/577

(54) **ANTIBODY AGAINST RESPIRATORY SYNCYTIAL VIRUS AND USE THEREOF**

(30) Priority: 11.06.2021 CN 202110653393
(71) Applicant: Institute of Microbiology, Chinese Academy of Sciences, Beijing 100101 (CN)
(72) Inventor: GAO, Fu, Beijing 100101 (CN); DAI, Lianpan, Beijing 100101 (CN); SONG, Jian, Beijing 100101 (CN); XU, Senyu, Beijing 100101 (CN); WANG, Qihui, Beijing 100101 (CN)
(74) Representative: Perani & Partners S.p.A.
(86) International application number: PCT/CN2022/098367
(87) International publication number: WO 2022/258068

(57) **Abstract**

Provided in the present disclosure is an antibody or an antigen-binding fragment thereof that binds to the respiratory syncytial virus F protein. Further provided in the present disclosure is the use of the antibody or the antigen-binding fragment thereof in the preparation of a drug for the treatment and/or prevention of RSV infections.

## Description

### CROSS-REFERENCE TO RELATED APPLICATION

The present application claims the benefit of a priority of Chinese Patent Application No. 202110653393.6, filed with the CNIPA on June 11, 2021, the entire contents of which are incorporated herein by reference.

### TECHNICAL FIELD

The present disclosure relates to an antibody or antigen-binding fragment thereof that binds to an F protein of respiratory syncytial virus. The present disclosure also relates to the use of the antibody or antigen-binding fragment thereof in the preparation of a medicament for the treatment and/or prevention of RSV infection.

### BACKGROUND

Respiratory syncytial virus (RSV) may cause acute lower respiratory tract infection, and it is the main pathogen causing illness and death of children all over the world. The hospitalization probability of RSV-related diseases is three times that of influenza or parainfluenza virus-related diseases. The high hospitalization rate is common in infants under 5 years old, and the prevalence rate is the highest in 3-year-old infants. RSV accounts for 6.7% among the deaths of infants aged from 1 month to 1 year in the world, and RSV is the most important pathogen of respiratory infection in preschool children, especially infants, and adults and the elderly with low immunity are also susceptible. Although RSV is a serious threat to people's health all over the world, there are few measures to prevent and treat RSV infection, and the research and development of vaccine is also hindered by the disease deterioration caused by formalin inactivated vaccine. Antibodies in the mother will be transmitted to the baby through the placenta in the last few weeks of pregnancy, which may provide protective immunity, but this protection will decline twice every month, and its durability is poor. At present, there is no vaccine and specific treatment. The only preventive measure is Palivizumab, which was approved in 1998. Although Palivizumab is capable of reducing the hospitalization rate and mortality rate of infants to a certain extent, it has poor immune activity and high frequency of use. Premature infants with congenital respiratory circulatory system disorders need to be immunized five times throughout the epidemic season. Such high cost and inconvenience make it unsuitable for all infants and limit the prevention of high-risk infants. Besides, a version of monoclonal antibody MEDI8897 produced by AstraZeneca, which is improved on monoclonal antibody D25, has strong neutralizing activity and only needs to be immunized for 1-2 times. Now it has entered the phase 3 of clinical trial. Therefore, a new generation of monoclonal antibody medicaments with high efficiency is urgently needed to prevent/treat RSV infection.

So far, neutralizing antibody has been proved to be an effective method to treat viral diseases. Currently marketed medicaments to treat and prevent viral infections include palivizumab (Synagis) to prevent pediatric respiratory syncytial virus (RSV) infection, Trogarzo to treat HIV infection, and Rabishield for the post-exposure prophylaxis of rabies virus. There are also a number of monoclonal antibodies that are at various stages of clinical research (https://clinicaltrials.gov/). Antibodies work mainly in two ways. On the one hand, antibodies having neutralizing activity may block the combination of virus with cell receptor by binding to a virus envelope protein, thereby blocking virus infection. On the other hand, antibody-dependent cell-mediated cytotoxicity (ADCC) and complement-dependent cytotoxicity (CDC) may recruit immune cells and immune molecules such as macrophages or complement, thereby eliminating free viruses and infected cells.

Respiratory syncytial virus (RSV) belongs to paramyxovirus family and is a single-stranded negative-strand RNA virus with two antigenic types, A and B, both of which are prevalent. Adsorption protein (G) and fusion protein (F) on the RSV surface are the main antigens capable of inducing neutralizing antibody response. G protein is the protein responsible for virus adhesion to the cell membrane surface, but it has antigenic variability, so it is difficult to produce a broad-spectrum protective medicament. Protein F is a protein responsible for the fusion of virus and cells, showing more neutralizing antibody targeting epitopes. It is the main target of most vaccines and immunotherapy medicaments under development, and also the target of preventing RSV diseases through palivizumab in clinic. Protein F has two conformations: pre-fusion and post-fusion. The pre-fusion F protein is unstable and may undergo conformational inversion to become the post-fusion F protein. So far, it has been found that most strongly neutralizing epitopes are concentrated in F protein in the pre-fusion conformation, such as monoclonal antibody D25 and its improved version of monoclonal antibody MEDI8897, which are bound to the ϕ epitope of pre-fusion F. In 2013, Peter Kwong's team obtained a stable pre-fusion RSV F protein DS-Cav1 by introducing a group of mutations (S190F, V207, S155C and S290C). Mice immunized with DS-Cav1 can activate higher production of neutralizing antibodies.

### SUMMARY

In one aspect, the present disclosure provides an antibody or antigen-binding fragment thereof that binds to an F protein of a respiratory syncytial virus, comprising a heavy chain variable region, wherein HCDR1, HCDR2 and HCDR3 of the heavy chain variable region are selected from one of the following groups:
1) an amino acid sequence of HCDR1 being GFTFSSYA (SEQ ID NO: 18);
   an amino acid sequence of HCDR2 being ISYDGSNT (SEQ ID NO: 19);
   an amino acid sequence of HCDR3 being ARDYCSRGTCYHDY (SEQ ID NO: 20);
2) an amino acid sequence of HCDR1 being GYTFTTYD (SEQ ID NO: 24);
   an amino acid sequence of HCDR2 being LNPDNGNT (SEQ ID NO: 25);
   an amino acid sequence of HCDR3 being TRAPWWWYFDY (SEQ ID NO: 26); and
3) an amino acid sequence of HCDR1 being GFSFTNYG (SEQ ID NO: 30);
   an amino acid sequence of HCDR2 being ISYDDGSDK (SEQ ID NO: 31);
   an amino acid sequence of HCDR3 being VRDPTGDY (SEQ ID NO: 32).

In some embodiments, the antibody or antigen-binding fragment thereof further comprises a light chain variable region, wherein HCDR1, HCDR2 and HCDR3 of the heavy chain variable region and LCDR1, LCDR2 and LCDR3 of the light chain variable region are selected from one of the following groups:
1) an amino acid sequence of LCDR1 being QDIRND (SEQ ID NO: 15);
   an amino acid sequence of LCDR2 being AAS (SEQ ID NO: 16);
   an amino acid sequence of LCDR3 being LQDYNYPQTFG (SEQ ID NO: 17);
   an amino acid sequence of HCDR1 being GFTFSSYA (SEQ ID NO: 18);
   an amino acid sequence of HCDR2 being ISYDGSNT (SEQ ID NO: 19);
   an amino acid sequence of HCDR3 being ARDYCSRGTCYHDY (SEQ ID NO: 20);
2) an amino acid sequence of LCDR1 being SGSIASNY (SEQ ID NO: 21);
   an amino acid sequence of LCDR2 being EDN (SEQ ID NO: 22);
   an amino acid sequence of LCDR3 being QSYDTSNAVFG (SEQ ID NO: 23);
   an amino acid sequence of HCDR1 being GYTFTTYD (SEQ ID NO: 24);
   an amino acid sequence of HCDR2 being LNPDNGNT (SEQ ID NO: 25);
   an amino acid sequence of HCDR3 being TRAPWWWYFDY (SEQ ID NO: 26); and
3) an amino acid sequence of LCDR1 being SLNIGSNY (SEQ ID NO: 27);
   an amino acid sequence of LCDR2 being KNN (SEQ ID NO: 28);
   an amino acid sequence of LCDR3 being AAWDDSLSGVVFG (SEQ ID NO: 29);
   an amino acid sequence of HCDR1 being GFSFTNYG (SEQ ID NO: 30);
   an amino acid sequence of HCDR2 being ISYDDGSDK (SEQ ID NO: 31);
   an amino acid sequence of HCDR3 being VRDPTGDY (SEQ ID NO: 32).

In some embodiments, in the antibody or antigen-binding fragment thereof according to any one of the foregoing, the heavy chain variable region comprises a sequence set forth in SEQ ID NO: 4, 6 or 8, or comprises an amino acid sequence having at least 90% sequence identity to the sequence set forth in SEQ ID NO: 4, 6 or 8.

In some embodiments, in the antibody or antigen-binding fragment thereof according to any one of the foregoing, the heavy chain variable region comprises a sequence set forth in SEQ ID NO: 4, or comprises an amino acid sequence having at least 90% sequence identity to SEQ ID NO: 4, and the light chain variable region comprises a sequence set forth in SEQ ID NO: 5, or comprises an amino acid sequence having at least 90% sequence identity to SEQ ID NO: 5; or
the heavy chain variable region comprises a sequence set forth in SEQ ID NO: 6, or comprises an amino acid sequence having at least 90% sequence identity to SEQ ID NO: 6, and the light chain variable region comprises a sequence set forth in SEQ ID NO: 7, or comprises an amino acid sequence having at least 90% sequence identity to SEQ ID NO: 7; or
the heavy chain variable region comprises a sequence set forth in SEQ ID NO: 8, or comprises an amino acid sequence having at least 90% sequence identity to SEQ ID NO: 8, and the light chain variable region comprises a sequence set forth in SEQ ID NO: 9, or comprises an amino acid sequence having at least 90% sequence identity to SEQ ID NO: 9.

In some embodiments, in the antibody or antigen-binding fragment thereof according to any one of the foregoing, the heavy chain constant region comprises a sequence set forth in SEQ ID NO: 10, or comprises an amino acid sequence having at least 90% sequence identity to SEQ ID NO: 10, and the light chain constant region of the antibody comprises a sequence set forth in SEQ ID NO: 11 or 12, or comprises an amino acid sequence having at least 90% sequence identity to SEQ ID NO: 11 or 12.

In some embodiments, the antibody or antigen-binding fragment thereof according to any one of the foregoing, wherein the antibody is a murine derived antibody, a chimeric antibody, a human derived antibody or a fully human derived antibodies; preferably, the antibody is a human derived antibody.

In some embodiments, the antibody or antigen-binding fragment thereof according to any one of the foregoing, wherein the F protein is in a pre-fusion conformation.

In some embodiments, the respiratory syncytial virus is a respiratory syncytial virus A or a respiratory syncytial virus B.

In another aspect, the present disclosure provides a nucleic acid molecule which encodes the antibody or antigen-binding fragment thereof according to any one of the foregoing. Specifically, the nucleic acid molecule encodes the light chain, heavy chain, light chain variable region or heavy chain variable region of the antibody according to any one of the foregoing. In another aspect, the present disclosure provides an expression vector comprising the nucleic acid molecule as described herein.

In another aspect, the present disclosure provides a host cell, which comprises or expresses the antibody or antigen-binding fragment thereof, or comprises the nucleic acid molecule or expression vector as described herein.

In another aspect, the present disclosure provides a pharmaceutical composition, which comprises the antibody or antigen-binding fragment thereof according to any one of the foregoing, and one or more pharmaceutically acceptable carriers, diluents, buffers or excipients.

In another aspect, the present disclosure provides use of the antibody or antigen-binding fragment thereof according to any one of the foregoing in the preparation of a medicament for a treatment and/or prevention of a respiratory syncytial virus-related disease or disorder. In another aspect, the present disclosure provides a method for treating and/or preventing respiratory syncytial virus-related diseases or disorders in a subject, comprising administering to a subject a therapeutically effective amount of the antibody or antigen-binding fragment thereof according to any one of the foregoing or the pharmaceutical composition as described herein.

Further, in the present disclosure, the respiratory syncytial virus-related disease or disorder according to any one of the foregoing is a disease or disorder related to upper respiratory tract infections or lower respiratory tract infections; more preferably, the disease or disorder is selected from tracheitis, bronchitis and pulmonary infectious diseases; most preferably, the disease or disorder is bronchitis or pneumonia.

In another aspect, the present disclosure provides a method for detecting a presence of a respiratory syncytial virus or an amount thereof in a sample, comprising: contacting the sample with the antibody or antigen-binding fragment thereof as described herein, and detecting a presence of a formation of an antigen-antibody complex or an amount of the formed antigen-antibody complex.

In another aspect, the present disclosure provides a kit for detecting a respiratory syncytial virus, comprising the antibody or antigen-binding fragment thereof according to any one of the foregoing.

### BRIEF DESCRIPTION OF THE DRAWINGS

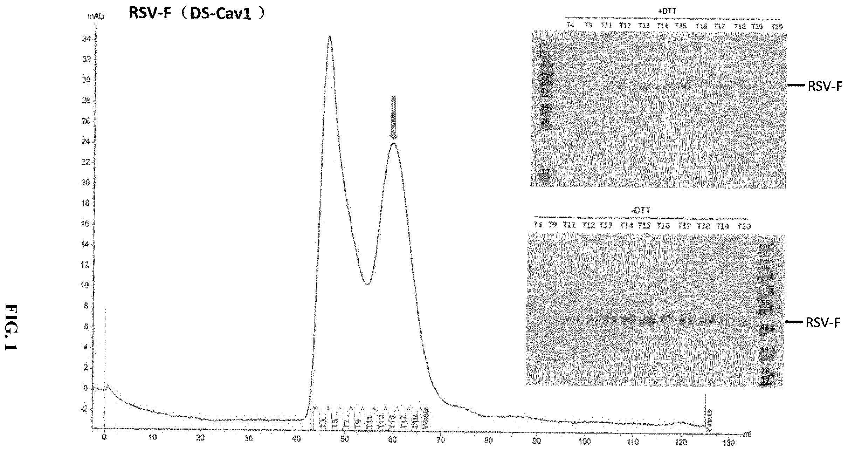

FIG. 1 shows the detection results of chromatography and SDS-PAGE for an expressed and purified RSV-F (DS-Cav1) protein.

### DETAILED DESCRIPTION

Unless otherwise specified, all technical and scientific terms used in the present disclosure have the meanings commonly understood by ordinary skilled persons in the art.

"Antibody" refers to immunoglobulin secreted by plasma cells (effector B cells) and used by the body's immune system to neutralize foreign substances (peptides, viruses, bacteria, etc.). The foreign substances are correspondingly called antigen. The term "antibody" is used in the broadest sense and covers various antibody structures, including but not limited to monoclonal antibodies, polyclonal antibodies; monospecific antibodies, multispecific antibodies (e.g., bispecific antibodies); full-length antibodies and antibody fragments (or antigen-binding fragments, or antigen-binding portions), as long as they exhibit desired antigen-binding activity. The basic structure of classical or common antibody molecules is a tetramer consisting of two identical heavy chains and two identical light chains. According to the conservative difference of amino acid sequences, the heavy chain and light chain are divided into variable regions (V) at amino terminal and constant regions (C) at carboxyl terminal. The variable regions of a heavy chain and a light chain interact to form an antigen binding site (Fv). In the variable region, the composition and arrangement order of amino acid residues in some regions are more liable to change than those in the other regions (framework region, FR) in the variable region, and these regions are called hypervariable regions (HVR). The hypervariable region is actually the key site of antibody binding to antigen. Because these hypervariable regions are complementary to antigenic determinants, they are also called complementarity determining regions (CDRs). Both heavy chain and light chain have three CDRs, which are called HCDR1, HCDR2, HCDR3 and LCDR1, LCDR2, LCDR3, respectively. Each VH and VL may be composed of three CDRs and four FR regions, which may be arranged in the following order from amino terminal to carboxyl terminal: FR1, CDR1, FR2, CDR2, FR3, CDR3 and FR4.

Given the sequence of the complementarity determining region of an antibody, a person skilled in the art may easily replace a part of the sequence of the antibody molecule (e.g., constant region or framework region) with a sequence from other species, for example, by DNA recombination technology, to form a chimeric antibody, and the chimeric antibody may basically retain the binding specificity of its source antibody. For example, where the source antibody is a murine antibody, its constant region may be replaced by a constant region of a human antibody, or conversely, where the source antibody is a human antibody, its constant region may be replaced by a constant region of a murine antibody, so as to weaken the immunogenicity of the antibody when it is used in different species or to make use of the specific functions of the constant region, such as ADCC-related activities. For purpose of further reducing the immunogenicity of the antibody or for other purposes, except the CDR sequence, all other the sequences in the antibody molecule may be replaced by a corresponding sequence of another antibody molecule (from the same or different species), which may comprise one or several amino acid mutations as appropriate, while basically retaining the binding specificity of the source antibody. In a specific example, persons skilled in the art often use CDR graft to humanize murine antibodies.

It may also be understood by those skilled in the art that on the basis of the specific antibody sequence provided in the present disclosure, a few amino acids may be substituted, deleted, added, and the binding ability of the obtained product with the corresponding antigen (F protein) or biological activity of the obtained product may be verified or screened, so as to obtain corresponding variants of the anti-F protein antibody molecule provided by the present disclosure, and these variants should also be included in the scope of the present disclosure.

Therefore, in some embodiments, the heavy chain variable region of the antibody molecule provided by the present disclosure comprises a sequence set forth in SEQ ID NO: 4 or an amino acid sequence having at least 85% (e.g., 86%, 87%, 88%, 89%, 90%, 91%, 92%, 93%, 94%, 95%, 96%, 97%, 98%, 99%) sequence identity to SEQ ID NO: 4, and the light chain variable region comprises a sequence set forth in SEQ ID NO: 5 or an amino acid sequence having at least 85% (e.g., 86%, 87%, 88%, 89%, 90%, 91%, 92%, 93%, 94%, 95%, 96%, 97%, 98%, 99%) sequence identity to SEQ ID NO: 5.

In some embodiments, the heavy chain variable region of the antibody molecule provided by the present disclosure comprises a sequence set forth in SEQ ID NO: 6 or an amino acid sequence having at least 85% (e.g., 86%, 87%, 88%, 89%, 90%, 91%, 92%, 93%, 94%, 95%, 96%, 97%, 98%, 99%) sequence identity to SEQ ID NO: 6, and the light chain variable region comprises a sequence set forth in SEQ ID NO: 7 or an amino acid sequence having at least 85% (e.g., 86%, 87%, 88%, 89%, 90%, 91%, 92%, 93%, 94%, 95%, 96%, 97%, 98%, 99%) sequence identity to SEQ ID NO: 7.

In some embodiments, the heavy chain variable region of the antibody molecule provided by the present disclosure comprises a sequence set forth in SEQ ID NO: 8 or an amino acid sequence having at least 85% (e.g., 86%, 87%, 88%, 89%, 90%, 91%, 92%, 93%, 94%, 95%, 96%, 97%, 98%, 99%) sequence identity to SEQ ID NO: 8, and the light chain variable region comprises a sequence set forth in SEQ ID NO: 9 or an amino acid sequence having at least 85% (e.g., 86%, 87%, 88%, 89%, 90%, 91%, 92%, 93%, 94%, 95%, 96%, 97%, 98%, 99%) sequence identity to SEQ ID NO: 9.

"Antigen-binding fragment" of an antibody refers to a polypeptide that comprises a partial sequence (especially a CDR sequence) of a source antibody and meanwhile has the binding specificity of the source antibody. The antigen-binding fragment usually comprises the light chain variable region and the heavy chain variable region of the source antibody, thus having antigen binding ability. There are many forms of antigen-binding fragments, such as Fab, F(ab')2, single chain Fv antibody (scFv), single domain antibody (sdAb) and so on. ScFv is composed of the heavy chain variable region and light chain variable region of the antibody, which are linked into a peptide chain through short peptides. By correct folding, the variable regions from heavy chain and light chain form Fv segments via non-covalent interaction, so scFv can better retain its affinity activity for antigens. SdAb was first discovered in camel, which naturally lacks light chain but still has antigen binding ability. Compared with ordinary antibody molecules, sdAb has some advantages, such as better stability (resistance to pH, heat and protease), and can target and bind epitopes on antigen molecules that are difficult for ordinary antibody molecules to reach.

"Specifically binding to an antigen" of an antibody or "antigen binding specificity" of an antibody mean that the antibody is capable of binding to a target antigen with higher binding affinity than the other antigens. The binding ability of the antibody to the target antigen may be qualitatively or quantitatively identified by various methods, such as determining the K_{D} value of the antibody binding to the target antigen, determining the ability of the antibody to competitively bind to the target antigen with other antibodies, etc. Here K_{D} is an equilibrium dissociation constant, which may be used to measure the binding affinity between the antibody and the antigen. The smaller the K_{D} value, the stronger the affinity. The specifical binding of an antibody to a target antigen does not mean that the antibody cannot bind to other antigens. For example, immune cross-reaction is also known in the art.

As used in the present disclosure, "antibody" may comprise an intact antibody or an antigen-binding fragment thereof, and may be a human antibody, a murine antibody, a humanized antibody, a chimeric antibody, etc. The antibody may be of any type, such as IgG, IgE, IgM, IgD, IgA or IgY), or any subclass, such as IgG1, lgG2, IgG3, lgG4, IgA1 or lgA2, etc.

Methods for preparing antibodies are known in the art, including but not limited to isolating cells expressing a specific antibody after immunizing animals with an antigen (for example, preparing an antibody by hybridoma technology), screening antibody technology by using phage antibody library, transforming cells by genetic engineering technology and making them express antibody molecules, etc. In a specific example, an example of the present disclosure provides a method for preparing antibody molecules by introducing recombinant expression vectors encoding antibody heavy chain and light chain into 293T cells.

The term "chimeric" antibody refers to an antibody in which a part of the heavy and/or light chain is derived from a specific source or species, and the rest of the heavy and/or light chain is derived from another different source or species.

The term "humanized" antibody is an antibody that retains the reactivity of a non-human antibody while having low immunogenicity in humans. For example, it may be achieved by retaining the non-human CDR region and replacing the rest of the antibody with its human counterpart (i.e., the constant region and framework region portions of the variable region).

Terms of "human antibody", "human derived antibody", "fully human antibodies" and "whole human antibody" may be used interchangeably, which refer to an antibody in which the variable region and the constant region are human sequences. These terms cover antibodies derived from a human gene but having altered sequences, such as sequences for reducing possible immunogenicity, increasing affinity, and eliminating cysteine or glycosylation sites that may cause undesirable folding. These terms cover antibodies produced by recombination in non-human cells, which may impart glycosylation that does not have the characteristics of human cells. The term also covers antibodies that have been bred in transgenic mice containing some or all of the human immunoglobulin heavy chain and light chain loci. The meaning of human antibody explicitly excludes humanized antibodies comprising non-human antigen binding residues.

The term "monoclonal antibody" refers to a group of substantially homogeneous antibodies, that is, the amino acid sequences of the antibody molecules comprised in the group are identical, except for natural mutations that may be present in small amount. In contrast, polyclonal antibody formulations typically comprise a variety of different antibodies having different amino acid sequences in their variable domains, which are typically specific to different epitopes. "Monoclonal" refers to the characteristics of antibodies obtained from the group of substantially homogeneous antibodies, and should not be interpreted as requiring the production of antibodies by any particular method. In some embodiments, the antibodies provided by the present disclosure are monoclonal antibodies.

The term "antigen" refers to a molecule or molecular part that may be bound by a selective binding agent such as an antigen-binding protein (including, for example, an antibody) and may additionally be used in animals to produce an antibody or its fragment capable of binding the antigen. An antigen may have one or more epitopes capable of interacting with different antigen-binding proteins (such as antibodies).

It is known in the art that antibody molecules may also be modified in many ways, such as PEGylation, glycosylation, formation of antibody molecule conjugates (such as ADC), purification labeling, or fusion with other proteins, e.g., forming bispecific antibodies. These antibody derivatives obtained by modification on the basis of antibody molecules provided in the present disclosure should also be included in the scope of the present disclosure.

When referring to amino acid sequences, the term "sequence identity" refers to the degree of identity between two amino acid sequences (such as a query sequence and a reference sequence), which is generally expressed as a percentage. Generally, sequence alignment is performed and gaps (if any) are introduced before calculating the percentage of identity between two amino acid sequences. If the amino acid residues in two sequences are identical at a certain alignment position, it is considered that the two sequences are consistent or matched at that position; if the amino acid residues in the two sequences are different, it is considered that they are inconsistent or mismatched at this position. In some algorithms, the number of matched positions is divided by the total number of positions in the comparison window to obtain a sequence identity. In other algorithms, the number of gaps and/or the length of gaps are also taken into account. For the purpose of the present disclosure, a disclosed alignment software BLAST (available on the web page ncbi.nlm.nih.gov) may be used to obtain the optimal sequence alignment and calculate the sequence identity between two amino acid sequences by using default settings.

"Expression vector", also known as "recombinant expression vector", refers to a nucleic acid molecule comprising various expression elements for expressing a target protein (e.g., a light chain and/or heavy chain of an antibody molecule) in a host cell. For an expression vector used to express a target protein in eukaryotic cells (mammalian cells, insect cells, plant cells, etc.), these expression elements usually include promoters, enhancers, polyadenylation signal sequences, etc. In order to facilitate amplification in *Escherichia coli,* the expression vector may also typically comprise an *Escherichia coli* replicon sequence. The expression vector may also comprise an antibiotic resistance gene or a selectable marker gene (such as ampicillin resistance gene (AmpR), thymidine kinase gene (TK), etc.) for screening and multiple cloning sites (MCS) for inserting an encoding sequence of a target protein. The expression vector may be a plasmid vector or a virus vector.

The expression vector provided in the present disclosure is suitable for expressing the antibody molecules or fragments thereof provided in the present disclosure in various host cells, especially in mammalian cells (such as cells of mice, rats, pigs, sheep, monkeys, orangutans, or human). In some embodiments, the mammalian cells may be selected from CHO cells, HEK293 cells or BHK cells.

In the present disclosure, using an F protein DS-Cav1 in the pre-fusion conformation of RSV as bait, four human monoclonal antibodies are isolated from the peripheral blood lymphocytes (PBMCs) of children recovered from RSV infection by single cell sequencing technology, which are named RV7, RV8, RV10 and RV11, respectively. Three of them are capable of binding to RSV F protein: RV8 and RV10 bind to both pre-fusion F protein and post-fusion F protein; RV11 only binds to pre-fusion F protein. These three monoclonal antibodies may be used for RSV diagnosis and antigen quantification. Two of them, RV8 and RV11, have neutralizing activity against both RSV A and RSV B and may be used for the prevention and treatment of RSV

The antibody or antigen-binding fragment thereof provided in the present disclosure may specifically bind to an F protein of respiratory syncytial virus (pre-fusion conformation and/or post-fusion conformation), that is, the F protein of respiratory syncytial virus is used as the target antigen of the antibody. After binding to the F protein of respiratory syncytial virus, the antibody inhibits the cell infection ability of RSV through neutralization activity. Therefore, the antibody or antigen-binding fragment thereof provided in the present disclosure may be used for preventing or treating RSV infection. On the other hand, because of the antigen binding specificity of the antibody provided in the present disclosure, the antibody may be used to detect the presence of RSV virus in a sample (such as blood) from a subject. Accordingly, the present disclosure provides an RSV detection kit that may comprise the antibody or antigen-binding fragment thereof provided in the present disclosure.

When referring to a pharmaceutical composition, the "pharmaceutically acceptable carrier" used refers to substances that may be safely administered, such as solid or liquid diluents, fillers, antioxidants, and stabilizers, and these substances are suitable for human and/or animal administration without excessive adverse side effects, and are also suitable for maintaining the activity of medicaments or active agents located therein.

"Subject" or "individual" includes human beings and non-human animals. The non-human animals include all vertebrates (e.g., mammals and non-mammals) such as non-human primates (e.g., Crab-eating Macaque), sheep, dogs, cows, chickens, amphibians and reptiles. Unless otherwise indicated, the term "patient" or "subject" is used interchangeably in the present disclosure. In certain embodiments, the individual or subject is a human.

"Administration", when applied to animals, humans, experimental subjects, cells, tissues, organs or biological fluids, refers to the contact of exogenous medicaments, therapeutic agents, diagnostic agents or compositions with animals, humans, subjects, cells, tissues, organs or biological fluids.

When referring to the treatment or prevention of RSV infection, "therapeutically effective amount" refers to the amount of active compound (such as an antibody) that is sufficient to cause biological or medical response expected by the clinician in a subject, and may also refer to the amount of medicament or drug that is sufficient to achieve or at least partially achieve the expected effect. The "therapeutically effective amount" of the antibody provided in the present disclosure may be determined by a person skilled in the art according to factors such as the route of administration, the weight, age and illness of the subject. For example, a typical daily dosage may range from 0.01 mg to 100 mg of an active ingredient per kg of body weight.

As used in the present disclosure, singular forms of "a", "an" and "the" include plural references, and vice versa, unless the context clearly indicates otherwise.

The present disclosure is further illustrated below through specific examples.

### Example 1: expression and purification of pre-fusion F protein (DS-Cav1) of RSV

The C-terminal of the protein encoding pre-fusion F protein DS-Cav1 of RSV A2 strain was added with a Thrombin enzyme-digestion site, six histidine sequences, one GS [glycine-serine] linker sequence, one sequence encoding a Strep II tag and one termination codon (amino acid sequence set forth in SEQ ID NO: 1), which was then inserted into EcoR I and XhoI enzyme-digestion sites of a mammalian expression vector pCAGGS. The ligation product was transformed into DH5α *Escherichia coli* competent cells. Then, monoclonals were picked out, inoculated into 4 mL LB medium, and cultured for 6-8 hours; then it was inoculated into 300 mL LB culture medium and cultured for 12-16 hours, and the bacteria were collected, and the plasmid was extracted by endotoxin-free plasmid extraction kit (TIANGEN) to obtain pCAGGS-RSV-F (DS-Cav1).

The extracted plasmid pCAGGS-RSV-F (DS-Cav1) was transfected into 293T cells to express F protein. 4-5 days after the transfection, the supernatant of 293T cell culture containing the target protein was centrifuged and filtered (0.22µM) to remove cell debris, then combined with a HisTrap HP (GE healthcare) nickel chelating column, and then the chromatographic column was eluted with different concentrations of imidazole, and the eluted proteins were collected, respectively. A sample containing the target protein was determined by SDS-PAGE results.

The elution peak containing the target protein was collected, concentrated, and subject to molecular sieve chromatography, and the size and purity of the target protein were determined according to the peak position of the protein and the SDS-PAGE results (see FIG. 1 for the results of molecular sieve and SDS-PAGE). The results of molecular sieve showed that the peak position of the protein was 60-70 mL, which was consistent with the theoretical molecular weight of F protein trimer of 150kDa. The SDS-PAGE results showed that the F protein was about ~50 Kda under both reduced (+DTT) and non-reduced (-DTT) conditions, suggesting that the F protein subunits formed trimers through non-covalent. The T-numbered sample of SDS-PAGE corresponded to the T-numbered collection tube of molecular sieve.

### Example 2: Isolation of RSV F (DS-Cav1) protein specific memory B cells

With the informed consent of the discharged person recovering after RSV infection, 3 to 10 mL of blood was collected and PBMCs were isolated. The isolated PBMCs (10⁷/mL) were incubated with 400 nM DS-Cav1 protein (prepared in Example 1) for binding for half an hour on ice, then washed twice with PBS and then mixed with the following antibodies (all purchased from BD): anti-human CD3/PE-Cy5, anti-human CD16/PE-Cy5, anti-human CD235a/PE-Cy5, anti-human CD19/APC-Cy7, anti-human CD27/Pacific Blue, anti-human CD38/APC, anti-human IgG/FITC and anti-His/PE. After incubation on ice for half an hour, the PBMCs were washed twice with PBS. Subsequently, the PBMCs were sorted using FACSAria III, and the cells of PE-CY5-APC-APC-CY7+Pacific Blue+FITC+PE+(that is, memory B cells) were collected directly into a 96-well plate, 1 cell/well.

### Example 3: single B cell PCR and cloning of human derived monoclonal antibody IgG1

The cells obtained in Example 2 were reverse transcribed by Superscript III Reverse Transcriptase (Invitrogen) at 55°C for 60 minutes. Using the above reverse transcription products as templates, antibody variable region sequences were amplified by PCR (PCRa) using HotStar Tap Plus enzyme (QIAgen). The reaction conditions were as follows: 95°C for 5 minutes; 95°C for 30 s, 55°C (heavy chain/ κ chain)/ 50°C (λ chain) for 30 s, 72°C for 90 s, 35 cycles; 72°C for 7min. The product obtained was used as a template for another round of PCR (PCRb) under the following conditions: 95°C for 5minutes; 95°C for 30 s, 58°C (heavy chain)/60°C (κ chain)/64°C (λ chain) for 30 s, 72°C for 90 s, 35 cycles; 72°C for 7min. The PCR products were isolated by 1.2% agarose gel electrophoresis. Gels with band between 400 and 500 bp were cut, recovered and sent to a sequencing company for sequencing. The sequencing results were analyzed on IGBLAST website. The analysis results were as follows.

Four pairs of antibodies were obtained: RV7, RV8, RV10 and RV11. According to the sequencing results, for RV7, the amino acid sequence of the heavy chain variable region was SEQ ID NO: 2, and the amino acid sequence of the light chain variable region was SEQ ID NO: 3; for RV8, the amino acid sequence of the heavy chain variable region of was SEQ ID NO: 4, and the amino acid sequence of the light chain variable region was SEQ ID NO: 5; for RV 10, the amino acid sequence of the heavy chain variable region was SEQ ID NO: 6; the amino acid sequence of the light chain variable region was SEQ ID NO: 7; for RV 11, the amino acid sequence of the heavy chain variable region was SEQ ID NO: 8, and the amino acid sequence of the light chain variable region was SEQ ID NO: 9. The light chain sequencing results of the four antibodies were compared with the germline genes, and it was determined that RV7 used CLx; RV8 used CLκ; RV 10 used CLλ; RV11 used CLλ.

In order to obtain human antibodies for subsequent evaluation, we designed the construction of all anti-IgG1. The strategy was as follows:
Heavy chain: CMV promoter-Hind III-signal peptide (SP)- heavy chain variable region (VH)- heavy chain constant region (CH)-Not I;
Light chain κ: CMV promoter-Hind III-signal peptide (SP)- light chain variable region (VK)- light chain constant region (CLκ)- Not I;
Light chain λ: CMV promoter-Hind III-signal peptide (SP)- light chain variable region (VL)- light chain constant region (CLλ)- Not I.

The analyzed correct variable region sequence was linked with the constant regions of corresponding heavy chain CH and light chain CLκ (or light chain CLλ) by bridge PCR and cloned into an expression vector KT351861 to obtain a recombinant plasmid containing encoding genes of specific antibody light and heavy chains, wherein the variable regions of light and heavy chains were linked into a vector containing constant regions by using enzyme-digestion sites Hind III and Not I. Wherein the amino acid sequence of the heavy chain constant region CH is set forth in SEQ ID NO: 10; the amino acid sequence of the light chain constant region CLκ is set forth in SEQ ID NO: 11; the amino acid sequence of the light chain constant region CLλ is set forth in in SEQ ID NO: 12; the amino acid sequence of the signal peptide (SP) is set forth in in SEQ ID NO: 13.

### Example 4: Expression and purification of monoclonal antibody

293T cells were cultured in DMEM containing 10% FBS. 293T cells were co-transfected with the recombinant expression vectors respectively encoding the heavy chain and light chain of the antibody obtained in Example 3. After transfection for 4-6 hours, the cell culture medium was changed to serum-free DMEM, and the culture was continued for 3 days. The supernatant was collected, and DMEM was added to continue the culture for 4 days. Then the supernatant was collected again.

The collected supernatant was centrifuged at 8000 rpm for 90 minutes, mixed with an equal volume of a buffer containing 20 mM sodium phosphate (pH 7.0), then filtered with a 0.22 µm filter membrane, and then loaded into a column prepacked with protein A or G (5 mL, GE Healthcare). The protein bound to the prepacked column was eluted with 100 mM glycine (pH 3.0). The eluted fraction was concentrated and then purified by molecular sieve chromatography. Subsequently, the purified target protein was detected by SDS-PAGE (reducing and non-reducing). In the SDS-PAGE under a non-reducing condition, the antibody showed a single band, and in the SDS-PAGE under a reducing condition, the disulfide bond in the Fc region of the antibody was opened, showing two bands, and the purity of the antibody was over 95%.

### Example 5 Detection of binding performance of the antibody

### (1) Construction and expression of RSV pre-fusion F protein (FΔFP)

The C-terminal of the protein encoding post-fusion F protein (FΔFP) of RSV A2 strain was added with a Thrombin enzyme-digestion site, six histidine sequences, one GS [glycine-serine] linker sequence, one sequence encoding a Strep II tag and one termination codon (amino acid sequence set forth in SEQ ID NO: 14), which was then inserted into NdeI and XhoI enzyme-digestion sites of a mammalian expression vector pCAGGS. The ligation product was transformed into DH5α *Escherichia coli* competent cells. Then, monoclonals were picked out, inoculated into 4 mL LB medium, and cultured for 6-8 hours; then it was inoculated into 300 mL LB culture medium and cultured for 12-16 hours, and the bacteria were collected, and the plasmid was extracted by endotoxin-free plasmid extraction kit (TIANGEN) to obtain pCAGGS-RSV-FΔFP.

The extracted plasmid pCAGGS-RSV-FΔFP was transfected into 293T cells to express F protein. 4-5 days after the transfection, the supernatant of 293T cell culture containing the target protein was centrifuged and filtered (0.22µM) to remove cell debris, then combined with a HisTrap HP (GE healthcare) nickel chelating column, and then the chromatographic column was eluted with different concentrations of imidazole, and the eluted proteins were collected, respectively. A sample containing the target protein was determined by SDS-PAGE results.

The elution peak containing the target protein was collected, concentrated, and subject to molecular sieve chromatography, and the size and purity of the protein were determined according to the peak position of the protein and the SDS-PAGE results.

### (2) Preparation and purification of intact Fab of monoclonal antibody

### A. Preparation of sample

The intact antibody protein was concentrated to 10 mg/ml or more, and the concentrated solution was changed (by 200 folds) to a sample buffer (20 mm Na₃PO₄, 10 mm EDTA, pH 7.0). The antibody after the solution exchange was concentrated to 20 mg/ml or more, to which a certain amount of digestion buffer was added (cysteine·HCl was added to the sample buffer to 20 mM, pH 7.0) for dilution, and the final concentration was 10mg/ml or more.

### B. Preparation of enzyme

0.5 mL of fully mixed coupling enzyme beans (Thermo, Prod # 20341) was sucked into a reaction container using a pipette with the tip cut off, which was centrifuged at 1000 g/min for 2 minutes to remove the preservation solution; the beans was washed with 3 mL of digestion buffer for 3 times, and the eluate was discarded. Then 0.5 mL of a digestion buffer, was added. The mixture was gently inverted and mixed evenly.

### C. Enzyme digestion

The reaction tube was plugged, to which concentrated antibody (about 10 mg) was added. The lid was tightened, and the gap was sealed with a sealing film. The reaction tube was fixed to a rotary mixer and incubated at 37°C overnight.

### D. Fab recovery

After enzyme digestion overnight, centrifugation was performed at 1000 g/minute for 5 minutes, and the lower liquid was retained; the beans were washed twice with 1 ml of Protein G binding buffer (20 mm Na₂PO₄, pH 7.0), and the lower liquid was recovered. The lower liquid in the above two steps were mixed, and then the concentration thereof was measured. The lower liquid was concentrated and passed through protein G; the eluate of the target protein was concentrated and changed to 1×PBS buffer, and further purified by a molecular sieve.

### (3) Evaluation of the binding ability of monoclonal antibody to F protein

In this example, Biacore 3K (Biacore Inc.) was used for surface plasmon resonance analysis. The specific steps were as follows:
First of all, the pre-fusion F-protein DS-Cav1 and the post-fusion F-protein FΔFP were immobilized on Fc2 and Fc4 channels (flow cell, Fc) of CM5 chip, respectively, by amino coupling. Then, the purified Fab proteins of RV7, RV8, RV10 and RV11 antibodies were bound by means of antibody capture. Palivizumab was used as a positive control. In addition, the Fabs of RV7, RV8, RV10, RV11 and Palivizumab antibodies were serially double-diluted with a solution of 20 mM HEPES, 150 mM NaCl and pH 7.4. Then, the serially diluted Fab passed through respective channels in turn (loading from low concentration in turn). The kinetic curves of the binding of Fabs of RV7, RV8, RV10, RV11 and Palivizumab antibodies to DS-Cav1 or FΔFP were recorded, and the kinetic constant K_{D} was calculated by Biaevaluation Software 3K (Biacore, Inc.). The affinity results are shown in Table 1.

**Table 1 Binding constants of antibody Fab fragments to pre-fusion F-protein DS-Cav1 and post-fusion F-protein FΔFP**

| Antibody mAbs | **Binding constant [K_{D}(nM)]** | |
|---|---|---|
| | DS-Cav1 | FΔFP |
| RV7 | Null^{a} | Null |
| RV8 | 25.6 | 17.5 |
| RV10 | 10.1 | 7.1 |
| RV11 | 7.2 | Null |
| Palivizumab | 11.4 | 4.4 |

| | | |
|---|---|---|
| ^{a}Null means no binding. | | |

Results analysis: RV7 did not bind to either the RSV pre-fusion F protein or the RSV post-fusion F protein. RV8 bound to both RSV pre-fusion F protein (K_{D} = 25.6 nM) and RSV post-fusion F protein (K_{D} = 17.5 nM). RV10 bound to both pre-fusion F protein (K_{D} = 10.1 nM) and post-fusion F protein (K_{D} = 7.11 nM). RV11 antibody could only bind to the RSV pre-fusion F protein (K_{D} = 7.18 nM) but not bind to the post-fusion F protein.

### Example 6: Detection of neutralizing activity of antibodies

The neutralizing activity of an antibody was detected using a flow cytometry staining method, of which the specific steps were as follows. In a nutshell, the purified antibody was diluted by gradient concentration, mixed with a virus. After being incubated for a period of time, the former was used to infect susceptible cells prepared the day before. Two days later, the cells were fixed and permeabilized, stained with a primary antibody targeting F protein, stained again with a labeled secondary antibody, and finally detected by flow cytometry to count the proportion of infected cells. Finally, the neutralizing activity of the antibody was obtained by calculation.

### Operating procedures:

### 1. Cell preparation

1) Hep2 cells were spread in a 24-well plate 12-16 hours in advance, cultured in a 10%DMEM medium, and antibody neutralization experiment was performed after the confluence was 70% or more the next day.
2) Dilution of antibodies: there were five antibodies, RV7, RV8, RV10, RV11 and Palivizumab. Each antibody was purified and filtered to a sterile condition. The antibodies were added to a 48-well plate for triple-fold gradient dilution, with at least 350 µL-400 µL in each well, diluted with 2% FBS DMEM medium.
3) Dilution of viruses: the viruses were taken out of a -80% refrigerator and thawed, diluted with 2% FBS DMEM culture medium according to an appropriate virus dilution multiple, and then added into the 48-well plate of the antibodies, 300 µL/ well. (RSV_A2 or RSV-Long or RSV-B: 450 µL diluted to 40 mL, and the final titer: 1.2×10⁴ TCID50/100 µL).
4) Incubation of viruses and antibodies:
   The diluted viruses and antibodies were mixed evenly in a molar ratio of 1:1, and incubated in a CO₂ incubator at 37°C for 1 hour.
5) Wash cells:
   The well plate just filled with Hep2 cells was taken out of the incubator 10 minutes in advance, the culture medium was sucked off with a pipette. The cells were washed once with PBS, and 500 µL PBS was added to each well.
6) Addition of the virus-antibody mixture: the PBS in the cells was sucked out, and the mixture of the virus and cell supernatant was added to the cells at 300 µL per well, and incubated at 37°C for 1 hour. (One gradient had two wells, and each plate had 11 gradients, and each plate had two virus wells for comparison.)
7) Fluid supplementation: after the incubation, the supernatant was sucked off, and 1 mL of DMEM medium containing penicillin-streptomycin mixed dual antibiotics solution and 2% FBS was added to each well. The cells were cultured in an incubator for about 48 hours.

### 2. flow cytometry detection

1) The infected cells were observed, and the cells were collected after appropriate cytopathic changes occurred.
2) Digestion of cells: the cell culture medium was removed, and the cells were washed once with PBS, followed by addition of 120 microliters of trypsin to each well, and digestion of the cells in an incubator at 37°C for 2minutes. After the cells fell off, the reaction was terminated with 10% FBS+ 130 µL PBS.
3) The cells were transferred to a 96-well plate having a round bottom. Centrifugation at 500 g was performed at 4°C for 10 minutes, and the supernatant was removed.
4) Each well was washed once with 150 µL of 1% FBS+PBS, centrifugation was performed as above, and the supernatant was removed.
5) 100 µL of a Fixation and Permeabilization Solution was added to fix and permeabilize the cells for 30minutes, kept away from light on ice at 4°C.
6) 100 µL of 1×wash buffer was added and mixed evenly. Centrifugation at 500 g was performed at 4°C for 10 minutes. 100 µL of wash buffer was used for re-suspension, a 96-well plate was sprayed with alcohol and sealed, and then it was transferred from P3 to P2 for subsequent operation. To be on the safe side, let it stand at 4°C overnight before operation. Or the cells were transferred to a new round-bottomed 96-well plate.
7) Addition of a primary antibody: the purified Palivizumab was diluted with 1×wash buffer to a concentration of 2 µg/mL, the cells were centrifuged, and the supernatant was removed. 50 µL of the antibody was added to each well, and incubated on ice for 30minutes. Subsequently, 150 µL of 1× wash buffer was added, mixed evenly, centrifuged, and the supernatant was removed.
8) Addition of a secondary antibody: Anti-Human-IgG (FITC) was diluted with 1×wash buffer, and the antibody was diluted at 1: 150 times. 50 µL of the antibody was added to each well, and incubated on ice for 30minutes. Subsequently, 150 µL of 1×wash buffer was added, mixed evenly, centrifuged, and the supernatant was removed. The washing was repeated once. Finally, re-suspension was performed with 150 µL of PBS.

Final flow cytometry detection: the flow cytometry analysis was carried out using BD FACSAriaII. FlowJo7.6.1 was used for analysis.

### Results

For RSV A-type A2 strain, the half inhibitory concentration IC50 of the antibody was 539.5 ng/mL for RV8 and 42.3 ng/mL for RV11, respectively, both of which were better than 751 ng/mL for palivizumab on the market, especially the RV11 antibody, whose neutralization activity was about 18 times stronger than that of palivizumab. Within the range of the detection concentration, RV7 and RV10 had no neutralization activity. See Table 2 for the data.

**Table 2 Neutralization activity of antibodies to RSV A-type A2 strain**

| **Antibody mAbs** | **IC50 (ng/mL)** |
|---|---|
| | RSV-A2 |
| RV7 | Null |
| RV8 | 539.5 |
| RV10 | Null |
| RV11 | 42.3 |
| Palivizumab | 751 |

| | |
|---|---|
| ^{a}Null means no neutralization activity. | |

For RSV A-type Long strain, the half inhibitory concentration IC50 of the antibody was 842.9 ng/mL for RV8 and 56.3 ng/mL for RV11, respectively, both of which were better than that (IC50=897.4 ng/mL) for palivizumab on the market, especially the RV11 antibody, whose neutralization activity was about 16 times stronger than that of palivizumab. Within the range of the detection concentration, RV7 had no neutralization activity. See Table 3 for the data.

**Table 3 Neutralization activity of antibodies to RSV A-type Long strain**

| **Antibody mAbs** | **IC50 (ng/mL)** |
|---|---|
| | RSV-Long |
| RV7 | Null^{a} |
| RV8 | 842.9 |
| RV11 | 56.3 |
| Palivizumab | 897.4 |

| | |
|---|---|
| ^{a}Null means no neutralization activity. | |

For RSV B-type clinical isolates, the half inhibitory concentration IC50 of the antibody was 183.1 ng/mL for RV8 and 31.4 ng/mL for RV11, respectively, both of which were better than that (IC50= 1413.0 ng/mL) for palivizumab on the market, especially the RV11 antibody, whose neutralization activity was about 45 times stronger than that of palivizumab. The neutralizing activity of RV8 antibody was also 7.7 times higher than that of Palivizumab. See Table 4 for the data.

**Table 4 Neutralization activity of antibodies to RSV B-type clinical isolates**

| **Antibody mAbs** | **IC50 (ng/mL)** |
|---|---|
| | RSV-Long |
| RV8 | 183.1 |
| RV11 | 31.4 |
| Palivizumab | 1413 |

Some amino acid sequences mentioned in the present disclosure are as follows:
SEQ ID NO: 1 
MELLILKANAITTILTAVTFCFASGQNITEEFYQSTCSAVSKGYLSALRTGWYTSVITIELS
NIKENKCNGTDAKVKLIKQELDKYKNAVTELQLLMQSTPATNNRARRELPRFMNYTL
NNTKKTNVTLSKKRKRRFLGFLLGVGSAIASGVAVCKVLHLEGEVNKIKSALLSTNKA
VVSLSNGVSVLTFKVLDLKNYIDKQLLPILNKQSCSISNIETVIEFQQKNNRLLEITREFS
VNAGVTTPVSTYMLTNSELLSLINDMPITNDQKKLMSNNVQIVRQQSYSIMCIIKEEVL
AYVVQLPLYGVIDTPCWKLHTSPLCTTNTKEGSNICLTRTDRGWYCDNAGSVSFFPQA
ETCKVQSNRVFCDTMNSLTLPSEVNLCNVDIFNPKYDCKIMTSKTDVSSSVITSLGAIVS
 
CYGKTKCTASNKNRGIIKTFSNGCDYVSNKGVDTVSVGNTLYYVNKQEGKSLYVKGE
PIINFYDPLVFPSDEFDASISQVNEKINQSLAFIRKSDELLGSGYIPEAPRDGQAYVRKDG
EWVLLSTFLLVPRGSHHHHHHGSWSHPQFGK
SEQ ID NO: 2 
QVQLQESGPGLVKPSETLSLTCTVSGGSISSYYLSWIRQPPGKGLEWIGYIYHTGSTNYN
PSLKSRVTISVHSSKNQFSLKLTSVTAADTAVYYCARHMRIVTTIDYWGQGTLVTVSS
SEQ ID NO: 3 
DIVMTQSPSSLSASVGDRVTLTCRASQGISSYLAWYQQKPGKVPKLLIYAASTLQSGVP
SRFSGSGSGTDFTLTISSLQPEDVATYYCQKYNSAPWTFGQGTKVEIK
SEQ ID NO: 4 
QVQLVQSGGGVVQPGRSLRLSCAASGFTFSSYAMHWVRQAPGKGLEWMAVISYDGS
NTYYADSVKGRFTISRDNSKNTLYLQMNSLRAEDTAMFYCARDYCSRGTCYHDYWG
QGTLVTVSS
SEQ ID NO: 5 
DIVLTQSPSSLSASVGDRVTITCRASQDIRNDLSWYQQKPGKAPKLLIYAASSLQSGVPS
RFSGSGSGTDFTLTISSLQPEDFATYSCLQDYNYPQTFGQGTKVEIK
SEQ ID NO: 6 
QVQLVQSGAEVKKPGASVKVSCKASGYTFTTYDFHWVRQAPGQGLEWIGWLNPDNG
NTKHSQKFQGRVTFTRDTSASTAFMELSSLRSEDTAVYYCTRAPWWWYFDYWGQGTL
VTVSS
SEQ ID NO: 7 
NFMLTQPHSVSESPGKTVTISCTRSSGSIASNYVQWCQQRPGSSPTTVIYEDNQRPSGVP
DRFSGSIDSSSNSASLTISGLRTEDEADYYCQSYDTSNAVFGGGTKLTVLGQPKA
SEQ ID NO: 8 
QVQLVQSGGGVVQPGRSLRLSCAASGFSFTNYGMHWVRQAPGKGLEWVAVISYDDG
SDKYYADSVKGRFTISRDNSKNTLYLQMNSLRAEDTAVYYCVRDPTGDYGDFPEQDG
YYYYYGMDVWGQGTTVTVSS
SEQ ID NO: 9 
QAVLTQPPSASGTPGQRVTISCSGSSLNIGSNYVYWYQQLPGTAPKFLIYKNNQRPSGV
PDRFSGSKSGTSASLAISGLRSEDEADYYCAAWDDSLSGVVFGGGTKLTVLGQPKA
SEQ ID NO: 10 
ASTKGPSVFPLAPSSKSTSGGTAALGCLVKDYFPEPVTVSWNSGALTSGVHTFPAVLQS
SGLYSLSSVVTVPSSSLGTQTYICNVNHKPSNTKVDKRVEPKSCDKTHTCPPCPAPELL
GGPSVFLFPPKPKDTLMISRTPEVTCVVVDVSHEDPEVKFNWYVDGVEVHNAKTKPR
EEQYNSTYRVVSVLTVLHQDWLNGKEYKCKVSNKALPAPIEKTISKAKGQPREPQVYT
LPPSRDELTKNQVSLTCLVKGFYPSDIAVEWESNGQPENNYKTTPPVLDSDGSFFLYSKL
TVDKSRWQQGNVFSCSVMHEALHNHYTQKSLSLSPGK
SEQ ID NO: 11 
RTVAAPSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGECS
SEQ ID NO: 12 
APSVTLFPPSSEELQANKATLVCLISDFYPGAVTVAWKADSSPVKAGVETTTPSKQSNN
KYAASSYLSLTPEQWKSHRSYSCQVTHEGSTVEKTVAPTECS
SEQ ID NO: 13
   ETDTLLLWVLLLWVPGSTGD
SEQ ID NO: 14

MELLILKANAITTILTAVTFCFASGQNITEEFYQSTCSAVSKGYLSALRTGWYTSVITIELS
NIKENKCNGTDAKVKLIKQELDKYKNAVTELQLLMQSTPATNNRARRELPRFMNYTL
NNAKKTNVTLSKKRKRRAIASGVAVSKVLHLEGEVNKIKSALLSTNKAVVSLSNGVSV
LTSKVLDLKNYIDKQLLPIVNKQSCSISNIETVIEFQQKNNRLLEITREFSVNAGVTTPVS
TYMLTNSELLSLINDMPITNDQKKLMSNNVQIVRQQSYSIMSIIKEEVLAYVVQLPLYG
VIDTPCWKLHTSPLCTTNTKEGSNICLTRTDRGWYCDNAGSVSFFPQAETCKVQSNRV
FCDTMNSLTLPSEVNLCNVDIFNPKYDCKIMTSKTDVSSSVITSLGAIVSCYGKTKCTAS
NKNRGIIKTFSNGCDYVSNKGVDTVSVGNTLYYVNKQEGKSLYVKGEPIINFYDPLVFP
SDEFDASISQVNEKINQSLAFIRKSDELLLVPRGSHHHHHHHHGSWSHPQFGK

## Claims

1. An antibody or antigen-binding fragment thereof that binds to an F protein of a respiratory syncytial virus comprising a heavy chain variable region, wherein HCDR1, HCDR2 and HCDR3 of the heavy chain variable region are selected from one of the following groups:
1) an amino acid sequence of HCDR1 being GFTFSSYA;
an amino acid sequence of HCDR2 being ISYDGSNT;
an amino acid sequence of HCDR3 being ARDYCSRGTCYHDY;
2) an amino acid sequence of HCDR1 being GYTFTTYD;
an amino acid sequence of HCDR2 being LNPDNGNT;
an amino acid sequence of HCDR3 being TRAPWWWYFDY; and
3) an amino acid sequence of HCDR1 being GFSFTNYG;
an amino acid sequence of HCDR2 being ISYDDGSDK;
an amino acid sequence of HCDR3 being VRDPTGDY

2. The antibody or antigen-binding fragment thereof according to claim 1, further comprising a light chain variable region, wherein HCDR1, HCDR2 and HCDR3 of the heavy chain variable region and LCDR1, LCDR2 and LCDR3 of the light chain variable region are selected from one of the following groups:
1) an amino acid sequence of LCDR1 being QDIRND;
an amino acid sequence of LCDR2 being AAS;
an amino acid sequence of LCDR3 being LQDYNYPQTFG;
an amino acid sequence of HCDR1 being GFTFSSYA;
an amino acid sequence of HCDR2 being ISYDGSNT;
an amino acid sequence of HCDR3 being ARDYCSRGTCYHDY;
2) an amino acid sequence of LCDR1 being SGSIASNY;
an amino acid sequence of LCDR2 being EDN;
an amino acid sequence of LCDR3 being QSYDTSNAVFG;
an amino acid sequence of HCDR1 being GYTFTTYD;
an amino acid sequence of HCDR2 being LNPDNGNT;
an amino acid sequence of HCDR3 being TRAPWWWYFDY; and
3) an amino acid sequence of LCDR1 being SLNIGSNY;
an amino acid sequence of LCDR2 being KNN;
an amino acid sequence of LCDR3 being AAWDDSLSGVVFG;
an amino acid sequence of HCDR1 being GFSFTNYG;
an amino acid sequence of HCDR2 being ISYDDGSDK;
an amino acid sequence of HCDR3 being VRDPTGDY

3. The antibody or antigen-binding fragment thereof according to claim 1 or 2, wherein the heavy chain variable region comprises a sequence set forth in SEQ ID NO: 4, 6 or 8, or comprises an amino acid sequence having at least 90% sequence identity to the sequence set forth in SEQ ID NO: 4, 6 or 8.

4. The antibody or antigen-binding fragment thereof according to claim 2 or 3, wherein the heavy chain variable region comprises a sequence set forth in SEQ ID NO: 4, or comprises an amino acid sequence having at least 90% sequence identity to SEQ ID NO: 4, and the light chain variable region comprises a sequence set forth in SEQ ID NO: 5, or comprises an amino acid sequence having at least 90% sequence identity to SEQ ID NO: 5; or
the heavy chain variable region comprises a sequence set forth in SEQ ID NO: 6, or comprises an amino acid sequence having at least 90% sequence identity to SEQ ID NO: 6, and the light chain variable region comprises a sequence set forth in SEQ ID NO: 7, or comprises an amino acid sequence having at least 90% sequence identity to SEQ ID NO: 7; or
the heavy chain variable region comprises a sequence set forth in SEQ ID NO: 8, or comprises an amino acid sequence having at least 90% sequence identity to SEQ ID NO: 8, and the light chain variable region comprises a sequence set forth in SEQ ID NO: 9, or comprises an amino acid sequence having at least 90% sequence identity to SEQ ID NO: 9.

5. The antibody or antigen-binding fragment thereof according to any one of claims 1 to 4, wherein a heavy chain constant region of the antibody comprises a sequence set forth in SEQ ID NO: 10, or comprises an amino acid sequence having at least 90% sequence identity to SEQ ID NO: 10, and a light chain constant region of the antibody comprises a sequence set forth in SEQ ID NO: 11 or 12, or comprises an amino acid sequence having at least 90% sequence identity to SEQ ID NO: 11 or 12.

6. The antibody or antigen-binding fragment thereof according to any one of claims 1 to 5, wherein the antibody is a human derived antibody.

7. The antibody or antigen-binding fragment thereof according to any one of claims 1 to 6, wherein the F protein is in a pre-fusion conformation.

8. The antibody or antigen-binding fragment thereof according to any one of claims 1 to 7, wherein the respiratory syncytial virus is a respiratory syncytial virus A or a respiratory syncytial virus B.

9. A nucleic acid molecule encoding the antibody or antigen-binding fragment thereof according to any one of claims 1 to 8.

10. An expression vector comprising the nucleic acid molecule according to claim 9.

11. A host cell comprising or expressing the antibody or antigen-binding fragment thereof according to any one of claims 1 to 8; or comprising the nucleic acid molecule according to claim 9; or comprising the expression vector according to claim 10.

12. A pharmaceutical composition comprising the antibody or antigen-binding fragment thereof according to any one of claims 1 to 8, and one or more pharmaceutically acceptable carriers, diluents, buffers or excipients.

13. Use of the antibody or antigen-binding fragment thereof according to any one of claims 1 to 8 in the preparation of a medicament for a treatment and/or prevention of a respiratory syncytial virus-related disease or disorder.

14. A method for treating and/or preventing respiratory syncytial virus-related diseases or disorders in a subject, comprising administering to a subject a therapeutically effective amount of the antibody or antigen-binding fragment thereof according to any one of claims 1 to 8 or the pharmaceutical composition according to claim 12.

15. The respiratory syncytial virus-related disease or disorder according to claim 13 or 14, wherein the respiratory syncytial virus-related disease or disorder is a disease or disorder related to upper respiratory tract infections or lower respiratory tract infections; preferably, the disease or disorder is selected from tracheitis, bronchitis and pulmonary infectious diseases; more preferably, the disease or disorder is bronchitis or pneumonia.

16. A method for detecting a presence of a respiratory syncytial virus or an amount thereof in a sample, comprising: contacting the sample with the antibody or antigen-binding fragment thereof according to any one of claims 1 to 8, and detecting a presence of a formation of an antigen-antibody complex or an amount of the formed antigen-antibody complex.

17. A kit for detecting a respiratory syncytial virus, comprising the antibody or antigen-binding fragment thereof according to any one of claims 1 to 8.
